**Europäisches Patentamt**

(19) **European Patent Office**

**Office européen des brevets**

(11) Veröffentlichungsnummer: **0 045 092**
**B1**

(12) **EUROPÄISCHE PATENTSCHRIFT**

(45) Veröffentlichungstag der Patentschrift:
03.07.85

(51) Int. Cl.⁴: **A 01 M 31/06**, A 23 K 1/16

(21) Anmeldenummer: 81106004.5

(22) Anmeldetag: 30.07.81

(54) **Köder zum Einfangen von wild lebenden Säugetieren, Verfahren zu ihrer Herstellung und ihre Verwendung.**

(30) Priorität: 30.07.80 HU 190480

(43) Veröffentlichungstag der Anmeldung:
03.02.82 Patentblatt 82/5

(45) Bekanntmachung des Hinweises auf die Patenterteilung:
03.07.85 Patentblatt 85/27

(84) Benannte Vertragsstaaten:
AT BE CH DE FR GB IT LI LU NL SE

(56) Entgegenhaltungen:
ANIMAL REGULATION STUDIES, Band 1, 1977, Seiten 23-46 Amsterdam, NL. A.M. HARTHOORN: "Problems relating to capture"
BIOLOGICAL ABSTRACTS, Band 68, Nr. 5, 1979, Seite 274, Auszug Nr. 27482 Philadelphia, Pa, U.S.A. F. GYULA: "Immobilization of Hungarias big game by means of a mixture of agents inducing a neuroleptanalgesic condition"

(73) Patentinhaber: **BUDAVIDEKI ALLAMI ERDO- ES VADGAZDASAG, H-2092 Budakeszi Pf. 74 (HU)**

(72) Erfinder: **Hönich, Miklós, Dr., Kisérleti Telep, H-2092 Budakeszi (HU)**

(74) Vertreter: **Beszédes, Stephan G. Dr., Münchener Strasse 80a Postfach 1168, D-8060 Dachau (DE)**

## Beschreibung

Die Erfindung betrifft neue Köder zum Einfangen von wild lebenden Säugetieren, ein Verfahren zu ihrer Herstellung und ihre Verwendung.

Zum Einfangen wild lebender Säugetiere wurde bisher mit der Injektionstechnik oder Umzäunungen gearbeitet. Diese Verfahren sind im Buch von Dr. E. Young B. V. «The Capture and Care of Wild Animals» ausführlich beschrieben. Beiden Verfahren ist als Nachteil gemeinsam, dass sie nicht genügend wirksam sind und ihre Anwendung sowohl für die Wildwirtschaft als auch für das lebende Wild gefährlich ist.

Das Einfangen des Wildes mittels Umzäunungen, das heisst im wesentlichen das Fangen des Wildes in einer Falle, ist nur nach vorhergehendem Füttern zum Hingewöhnen des Wildes, meistens im Winter, erfolgreich. In vielen Fällen vertragen sich die einzelnen Wildarten im eingezäunten Gebiet nicht, auch ist die Sterblichkeit bedeutend (bei einzelnen Tierarten 20 bis 50%). Nachteilig ist ferner, dass das umzäunte Gebiet ortsgebunden ist und seine Anlage und Aufrechterhaltung einen beträchtlichen Aufwand erfordert.

Die Injektionstechnik ist zwar weniger kostenaufwendig, dafür jedoch um so arbeits- und zeitaufwendiger, und es wird immer jeweils nur ein einziges Tier gefangen. Die Anwendung der Injektionstechnik ist ferner dadurch beschränkt, dass die verwendeten Wirkstoffe, zum Beispiel Succinylcholinchlorid, d-Tubocurare und Xylazinhydrochlorid, zum überwiegenden Teil Rauschgifte sind, die nur den dazu Berechtigten, das heisst Ärzten und Tierärzten, zugänglich sind und nur von diesen gehandhabt werden dürfen. Infolge unrichtiger Wahl der Dosis oder einer individuellen Überempfindlichkeit des Tieres kann die Sterblichkeit auch bei diesem Verfahren bedeutend sein.

Über einige Versuche zum Einfangen von wild lebenden Säugetieren durch mit dem Futter aufnehmbare Wirkstoffe enthaltende Köder wurde bereits früher berichtet. Nach dem Fachschrifttum (Brit. Vet. J. 131 [1975], 545; J. Wildl. Mgm. 31 [1967], 686; Der praktische Tierarzt 12/76, 830) wurde als Wirkstoff 7-Chlor-1-methyl-5-phenyl-3H-1,4-benzodiazepin-2(1H)-on (Diazepam, Valium®, Seduxen®) verwendet. Zwar wurden mit diesem Verfahren gute Erfolge erzielt, wenn den an einem eingegrenzten Ort gehaltenen Tieren der Köder auf Grund einer individuellen Schätzung des Körpergewichtes in vorher bestimmter Dosis verabreicht wurde, es ist jedoch zum Einfangen von frei lebendem Wild weniger geeignet. Wenn das Tier zu viel vom Köder frisst, kann es zugrundegehen; ansonsten ist das Tier zwar durch die Wirkung des 7-Chlor-1-methyl-5-phenyl-3H-1,4-benzodiazepin-2(1H)-ones betäubt, jedoch immer noch fähig, sich zur Wehr zu setzen oder zu fliehen. Das Einfangen ist daher schwierig. Gemäss Der praktische Tierarzt 12/76, 830 konnten die frei lebenden, mit dem 7-Chlor-1-methyl-5-phenyl-3H-1,4-benzodiazepin-2(1H)-on enthaltenden Köder gefütterten Tiere nur durch zusätzliche Anwendung der Injektionstechnik (mit Xylazinhydrochlorid) eingefangen werden.

Der Erfindung liegt die Aufgabe zugrunde, Köder zum Einfangen von wild lebenden Säugetieren, aus welchen die Wirkstoffe durch das Maul in den Organismus der Tiere gelangen und mit welchen das lebende Wild ohne vorherige Investitionen praktisch zu jedem beliebigen Zeitpunkt und an jedem beliebigen Ort eingefangen werden kann, ein Verfahren zu ihrer Herstellung und ihre Verwendung, durch welche das Einfangen von wild lebenden Säugetieren unabhängig von den Gegebenheiten des Reviers, den Jahreszeiten und der Tatsache, dass sich die einzelnen Wildarten durch ihr Verhalten gegenseitig stören, bewerkstelligt werden kann und welche auch von durchschnittlich ausgebildeten Fachkräften der Wildwirtschaft mit hoher Wirksamkeit vorgenommen werden kann, das heisst ohne spezielle Fachkenntnisse und ohne die Notwendigkeit von Investitionen optimale Ergebnisse erzielt werden können, zu schaffen.

Das Obige wurde überraschenderweise durch die Erfindung erreicht.

Es wurde nämlich überraschenderweise festgestellt, dass die wild lebenden Säugetiere kurze Zeit nach der Futteraufnahme zur Verteidigung unfähig werden und in der Nähe des Fütterungsortes leicht eingefangen werden können und gleichzeitig die Gefahr einer Vergiftung infolge Überdosierung auf ein Minimum verringert werden kann, wenn die Tiere mit Ködern, die auf ihrer Oberfläche ein Gemisch aus

a) 35 bis 95 Gew.-% von 1 oder mehr Schlafmitteln (n) unter Ausschluss von Barbituraten und/oder schwächer wirkenden Tranquillantium beziehungsweise Tranquillantia (schwächer wirkenden Ataracticum beziehungsweise Ataractica) [Minortranquillantium beziehungsweise Minortranquillantia] und

b) 5 bis 35 Gew.-% von 1 oder mehr stark wirkenden Tranquillantium beziehungsweise Tranquillantia (stark wirkenden Ataracticum beziehungsweise Ataractica) [Majortranquillantium beziehungsweise Majortranquillantia] und gegebenenfalls

c) bis zu 20 Gew.-% von 1 oder mehr Substanz(en) mit Antihistaminwirkung und/oder gegebenenfalls

d) bis zu 10 Gew.-% von 1 oder mehr Antiepilepticum beziehungsweise Antiepileptica, bezogen auf das Gewicht des Futterstoffes beziehungsweise der Futterstoffe, aufweisen, gefüttert werden. Die physiologische Grundlage der günstigen Wirkung besteht darin, dass einzelne Bestandteile dieser Wirkstoffkombination auch durch die Schleimhaut der Mundhöhle absorbiert werden und die Wirkung dieser Bestandteile durch die Wirksamkeit der im Magen absorbierten weiteren Wirkstoffbestandteile der Wirkstoffkombination ergänzt beziehungsweise verstärkt wird. Durch die auf diese Weise sich ergebende Wirkung, die letzten Endes einen dem biologischen Schlaf ähnlichen Zustand hervorruft, gelangt das Tier innerhalb kurzer Zeit in einen Zustand, in dem es nicht mehr fähig ist, mehr von dem Köder zu fressen (dadurch wird die Überdosierung vermieden) und andererseits infolge Absinkens des Blutdruckes, eines gewissen Angstgefühles und des Gefühles körperlicher Schwäche auch nicht mehr fähig ist, den Ort der Futteraufnahme zu verlassen, so dass es innerhalb eines kleinen Gebietes eingefangen werden kann.

Eigene Versuche ergaben, dass bei Verwendung der Bestandteile der obigen Wirkstoffkombination einzeln eine derart starke Betäubung, welche das Einfangen innerhalb eines kleinen Gebietes ermöglichen würde, nur mit Wirkstoffdosen, welche schon nahe bei der toxischen Dosis liegen, erreicht werden kann. Die einzelnen Bestandteile sind demnach für sich allein angewandt zum Einfangen von lebendem Wild ungeeignet. Ferner wurde festgestellt, dass in der Wirkstoffkombination der obigen Zusammensetzung die Antiepileptica, wie Barbiturate, die Toxizität der übrigen Bestandteile vermindern.

Gegenstand der Erfindung sind daher Köder zum Einfangen von wild lebenden Säugetieren gegebenenfalls mit einem Gehalt an einem schwächer wirkenden Tranquillantium, welche dadurch gekennzeichnet sind, dass sie 1 oder mehr als Futter für das Wild geeignete[n] Futterstoffe(e) und auf der Oberfläche des Futterstoffes beziehungsweise der Futterstoffe mindestens 0,5 Gew.-% einer Wirkstoffkombination, bestehend aus

a) 35 bis 95 Gew.-% von 1 oder mehr Schlafmittel(n) unter Ausschluss von Barbituraten und/oder schwächer wirkenden Tranquillantium beziehungsweise Tranquillantia (schwächer wirkenden Ataracticum beziehungsweise Ataractica) [Minortranquillantium beziehungsweise Minortranquillantia] und

b) 5 bis 35 Gew.-% von 1 oder mehr stark wirkenden Tranquillantium beziehungsweise Tranquillantia (stark wirkenden Ataracticum beziehungsweise Ataractica) [Majortranquillantium beziehungsweise Majortranquillantia] und gegebenenfalls

c) bis zu 20 Gew.-% von 1 oder mehr Substanz(en) mit Antihistaminwirkung und/oder gegebenenfalls

d) bis zu 10 Gew.-% von 1 oder mehr Antiepilepticum beziehungsweise Antiepileptica, bezogen auf das Gewicht des Futterstoffes beziehungsweise der Futterstoffe, sowie gegebenenfalls 1 oder mehr an sich bekannte[n] Hilfsstoff(e), zweckmässig Träger, die Haftung der Wirkstoffkombination, den Geschmack und/oder die Witterungsbeständigkeit verbessernde[n] Stoff(e) und/oder Farbstoff(e) enthalten.

Der Ausdruck «wild lebende Säugetiere» ist in seinem weitesten Sinne, also auch als von Tiergärten und Zirkussen ausgebrochene Tiere, wie Affen, Tiger und Wölfe, umfassend zu verstehen.

Vorzugsweise beträgt in den erfindungsgemässen Ködern die Menge der Wirkstoffkombination höchstens 10 Gew.-%, bezogen auf das Gewicht des Futterstoffes beziehungsweise der Futterstoffe.

In der auf der Oberfläche der erfindungsgemässen Köder befindlichen Wirkstoffkombination sind die Schlafmittel und die schwächer wirkenden Tranquillantia gegeneinander austauschbare Bestandteile; der Köder kann also nur den einen oder nur den anderen dieser Bestandteile enthalten, in anderen Fällen dagegen ein Gemisch von beiden. Im letzteren Fall beträgt das Gewichtsverhältnis des Schlafmittels beziehungsweise der Schlafmittel zum schwächer wirkenden Tranquillantium beziehungsweise zu den schwächer wirkenden Tranquillantien vorzugsweise 1 : 3 bis 3 : 1.

Vorzugsweise enthält die Wirkstoffkombination der erfindungsgemässen Köder 5 bis 15 Gew.-% stark wirkendes Tranquillantium beziehungsweise stark wirkende Tranquillantia.

Wie aus der festgelegten Zusammensetzung hervorgeht, ist ein Gehalt an Substanzen mit Antihistaminwirkung in den erfindungsgemässen Ködern nicht unbedingt erforderlich. Es ist jedoch bevorzugt, dass im Köder auch mindestens 1 Substanz mit Antihistaminwirkung vorliegt. In erfindungsgemässen Ködern, welche auch 1 oder mehr Substanz(en) mit Antihistaminwirkung enthalten, beträgt der Mengenanteil dieser Substanz(en) zweckmässig 4 bis 13 Gew.-% der Wirkstoffkombination.

Auch ein Gehalt an Antiepileptica ist nicht unbedingt erforderlich. Im Hinblick auf die die Toxizität verringernde Wirkung der Antiepileptica, wie Barbiturate, ist es jedoch zweckmässig, dass zumindest in denjenigen Ködern, welche eine grössere Wirkstoffkombinationsmenge, wie etwa 3 bis 10 Gew.-%, enthalten, auch 1 oder mehr Antiepileptica zugegen ist beziehungsweise sind. Das Antiepilepticum beziehungsweise die Antiepileptica ist beziehungsweise sind zweckmässig in Mengen von 1 bis 5 Gew.-% der Wirkstoffkombination enthalten.

Hervorragend günstige Eigenschaften haben diejenigen erfindungsgemässen Köder, welche sämtliche, das heisst 4 oder 5 Wirkstoffe der Wirkstoffkombination (Schlafmittel und/oder schwächer wirkendes Tranquillantium beziehungsweise schwächer wirkende Tranquillantia + stark wirkendes Tranquillantium beziehungsweise stark wirkende Tranquillantia + Substanz(en) mit Antihistamwirkung + Antiepilepticum beziehungsweise Antiepileptica) enthalten.

Die zweckmässige obere Grenze der Menge der Wirkstoffkombination, die in den erfindungsgemässen Ködern enthalten ist, hängt von der Art des Wildes, das eingefangen werden soll, ab. Erfindungsgemässe Köder zum Einfangen von Wildarten, welche die Wirkstoffe weniger vertragen, insbesondere Wiederkäuern, wie Damhirschen, Mufflonen und Rehen, enthalten zweckmässig höchstens 7 Gew.-% Wirkstoffkombination, bezogen auf das Gewicht des Futterstoffes beziehungsweise der Futterstoffe, während erfindungsgemässe Köder zum Einfangen von wild lebenden Säugetieren mit einer grösseren Toleranzfähigkeit, insbesondere den keinen zusammengesetzten Magen oder anders ausgedrückt einen ungeteilten Magen aufweisenden Wildarten, wie Wildschweinen und Füchsen, zweckmässig höchstens 10 Gew.-% Wirkstoffkombination, bezogen auf das Gewicht des Futterstoffes beziehungsweise der Futterstoffe, enthalten. Vorzugsweise enthalten die erfindungsgemässen Köder die Wirkstoffkombination in einer Menge von 1 bis 5 Gew.-%, insbesondere 2 bis 4 Gew.-%, bezogen auf das Gewicht des Futterstoffes beziehungsweise der Futterstoffe.

Die in der Wirkstoffkombination der erfindungsgemässen Köder enthaltenen beziehungsweise gegebenenfalls enthaltenen Schlafmittel, schwächer wirkenden Tranquillantia, stark wirkenden Tranquillantia, Stoffe mit Antihistaminwirkung beziehungsweise Antiepileptica können zum Beispiel die folgenden sein:

Schlafmittel: 1,3-Dihydro-7-nitro-5-phenyl-2H-1,4-benzodiazepin-2-on (Eunoctin®, Nitrazepam), α-Äthyl-α-phenylglutarsäureimid [3-Äthyl-3-phenylpiperidin-2,6-dion] (Noxyron®, Glutethimid) und 2-Methyl-4-(2',2',2'-trichlor-1'-hydroxyäthoxy)-propan-2-ol (Mechloral).

Schwächer wirkende Tranquillantia: 2-Methyl-2-n-propyl-n-propan-1,3-dioldicarbamat [2-Äthyl-2-propyl-1,3-propandioldicarbamat {Merck-Index, 9. Ausgabe}] (Meprobamat, Andaxin®), 7-Chlor-2-methylamino-5-phenyl-3H-1,4-benzodiazepin-4-oxyd (Elenium), 7-Chlor-1,3-dihydro-1-methyl-5-phenyl-2H-1,4-benzodiazepin-2-on (Diazepam, Seduxen®) und 1-(3,4-Dimethoxyphenyl)-4-methyl-5-äthyl-7,8-dimethoxy-5H-2,3-benzodiazepin (Grandaxin®).

Stark wirkende Tranquillantia: Phenothiazinderivate, wie 2-Chlor-10-(3'-dimethylamino-n-propyl)-phenothiazin (Hibernal®) und Ester von N-(β-Hydroxyäthyl)-N'-[3-(3'-γ-chlorphenothiazinyl)-n-propyl]-piperazindifumarat oder diäthylsulfonat mit Trimethoxybenzoesäure (Frenolon®).

Substanz mit Antihistaminwirkung: 10-(2-Dimethylamino-n-propyl)-phenothiazin (Pipolphen®).

Antiepileptica: Barbiturate, wie 5-(1'-Cyclohexenyl)-1,5-dimethylbarbitursäure (Hexobarbital), 5,5-Diallylbarbitursäure (Allobarbital) und 5-n-Butyl-5-äthylbarbitursäure (Butobarbital) sowie ihre Salze.

Obwohl die Barbiturate bekanntlich auch eine schlaffördernde Wirkung haben, wird im vorliegenden Text der Ausdruck «Schlafmittel» nicht auf die Barbiturate bezogen. Entsprechend ihrem zweiten Hauptwirkungsgebiet werden die Barbiturate hier als Antiepileptica betrachtet.

Vorzugsweise enthalten die erfindungsgemässen Köder als Hilfsstoff Adeps solidus (Hartfett), insbesondere ein solches von Arzneibuchqualität. Zweckmässig liegt dieses in einer Menge von 0,1 bis 120 Gew.-%, bezogen auf das Gewicht der Wirkstoffkombination, vor.

Vorteilhafte die Haftung der Wirkstoffkombination der erfindungsgemässen Köder verbessernde Stoffe beziehungsweise Haftvermittler für diese sind verschiedene Zucker, wie Traubenzucker, Rübenzucker und Fruchtzucker; diese Stoffe dienen gleichzeitig als den Geschmack verbessernde Stoffe. Die Haftung der Wirkstoffkombination der erfindungsgemässen Köder verbessernde Stoffe können jedoch auch andere bekannte Stoffe, zum Beispiel Gelatine oder Gummi arabicum, sein. Die Witterungsbeständigkeit verbessernde Stoffe oder anders ausgedrückt Stoffe zur Verbesserung der Widerstandsfähigkeit gegen Witterungseinflüsse der erfindungsgemässen Köder sind zweckmässig Substanzen, welche bei Zimmertemperatur fest sind, bei der Körpertemperatur des Wildes (36 bis 38 °C) jedoch schmelzen. Diese Stoffe liegen zweckmässig als äusserste Schicht auf dem Köder vor. Als die Witterungsbeständigkeit verbessernder Stoff ist Adeps solidus der in den Arzneibüchern vorgeschriebenen Qualität besonders geeignet. Das Adeps solidus kann aber auch der Träger der Wirkstoffkombination sein. Das Adeps solidus übt im Pansen der Wiederkäuer eine schaumzerstörende Wirkung aus und setzt dadurch das Auftreten von etwaigen unerwünschten Nebenwirkungen wesentlich herab. Der Mengenanteil von Adeps solidus spielt hier keine entscheidende Rolle. Beim Vorliegen des Adeps solidus in Mischung mit der Wirkstoffkombination beträgt seine Menge zweckmässig 0,1 bis 3,0 Gew.-%, bezogen auf das Gewicht der Wirkstoffkombination. Im Falle des Vorliegens des Adeps solidus auf dem mit der Wirkstoffkombination überzogenen Köder als äusserster Schicht beträgt seine Menge zweckmässig 0,1 bis 3,0 Gew.-%, bezogen auf das Gewicht des fertigen Köders, entsprechend 4 bis 120 Gew.-%, bezogen auf das Gewicht der Wirkstoffkombination. Die Farbstoffe der erfindungsgemässen Köder können nicht-toxische Farbstoffe, zum Beispiel die in der Lebensmittelindustrie zugelassenen Speisefarbstoffe, sein.

Vorzugsweise enthalten die erfindungsgemässen Köder als Futterstoff(e) körniges Futter, insbesondere Getreidekörner, wie Mais- und/oder Weizenkörner, und/oder granuliertes Futter. Vor allem zum Einfangen von Raubtieren können sie aber auch durch den Fleischwolf gedrehtes Fleisch und/oder ein aus Fleisch bereitetes Futter als Futterstoff(e) enthalten. Zum Einfangen von Wiederkäuern hat sich als Futterstoff Mais (ungeschrotet, das heisst in Kornform) besonders bewährt.

Gegenstand der Erfindung ist auch ein Verfahren zur Herstellung der erfindungsgemässen Köder, welches dadurch gekennzeichnet ist, dass in an sich bekannter Weise in 1 oder mehr Schritten die Wirkstoffkombination und gegebenenfalls der beziehungsweise die Hilfsstoff(e) auf die Oberfläche des Futterstoffes beziehungsweise der Futterstoffe aufgebracht wird beziehungsweise werden.

Nach einer bevorzugten Ausführungsform des erfindungsgemässen Verfahrens werden die einzelnen Wirkstoffe der Wirkstoffkombination sowie ferner 1 oder mehr den Geschmack und die Haftung verbessernder beziehungsweise verbessernde Stoff(e) und gegebenenfalls Farbsoff(e) und/oder Träger unter Erwärmen in Alkohol oder wässrigem Alkohol, zum Beispiel 80%-igem Äthanol, gelöst und die Lösung wird in bekannter Weise, zum Beispiel durch Giessen, Sprühen oder Zerstäuben, auf die Oberfläche des Futterstoffes beziehungsweise der Futterstoffe aufgebracht. Das so behandelte Futter wird trocknen gelassen. Danach kann beziehungsweise können auf seine Oberfläche, falls es erwünscht ist, als weitere Schicht 1 oder mehr die Witterungsbeständigkeit erhöhende[r] Stoff(e) und gegebenenfalls Träger aufgebracht werden.

Vorzugsweise wird im Falle der Verwendung von Adeps solidus als Träger und/oder die Witterungsbeständigkeit verbesserndem Hilfsstoff dieses in einem gesonderten Schritt auf die Oberfläche des beziehungsweise der die Wirkstoffkombination enthaltenden Futterstoffe[s] aufgebracht.

Ferner ist Gegenstand der Erfindung die Verwendung der erfindungsgemässen Köder zum Einfangen von wild lebenden Säugetieren durch Auslegen dieser Köder an Stellen, an welchen das Wild Nahrung zu sich nimmt, und sonstigen von ihm öfter aufgesuchten Stellen, wie Futterstellen, Wildwechsel und Wiesen, mit drauffolgendem Einsammeln des schlafenden oder betäubten Wildes an der Stelle des ausgelegten Köders oder in deren Umgebung.

Zweckmässig wird das Auslegen der Köder in der Wildart entsprechenden Portionen, beim Fangen von Grosswild zweckmässig in Portionen von 50 bis 300 g, insbesondere 100 bis 300 g, vorgenommen.

Die Tiere verzehren den ausgelegten Köder meistens in den Nachmittags- oder Abendstunden oder in der Nacht. Danach legen sie sich entweder unmittelbar an der Stelle des ausgelegten Köders oder in einem Umkreis von 10 bis 300 m davon nieder und geraten für 5 bis 20 Stunden in einen dem natürlichen Schlaf ähnlichen Zustand. Die schlafenden beziehungsweise betäubten Tiere werden zweckmässig 1 bis 5 Stunden nach dem Verzehr des Köders eingesammelt; sie werden im Interesse ihrer Sicherheit zweckmässig an einen geschlossenen Ort gebracht. Es muss darauf geachtet werden, dass beim Transport und danach die schlafenden beziehungsweise betäubten Tiere eine natürliche liegende Körperhaltung einnehmen.

Die Erfindung wird an Hand der folgenden Beispiele näher erläutert.

Beispiel 1

Es wurde eine Wirkstoffkombination der folgenden Zusammensetzung verwendet:

40 g 1,3-Dihydro-7-nitro-5-phenyl-2H-1,4-benzo-diazepin-2-on

40 g 7-Chlor-1,3-dihydro-1-methyl-5-phenyl-2H-1,4-benzodiazepin-2-on

10 g 10-(2-Dimethylamino-n-propyl)-phenothiazin

10 g 2-Chlor-10-(3'-dimethylamino-n-propyl)-phenothiazin

5 g 5-(1'-Cyclohexenyl)-1,5-dimethylbarbitur-säure

Die Bestandteile des Wirkstoffgemisches wurden miteinander vermischt, zum Gemisch wurden 200 cm³ 80%-iger wässriger Äthylalkohol zugegeben und die Wirkstoffe wurden bei 80 °C in diesem Lösungsmittel gelöst. Die Lösung wurde mit Traubenzucker gesättigt (dazu waren etwa 20 g Traubenzucker erforderlich). Dann wurde die heisse Lösung auf 5 kg Maiskörner, die vorher auf 30 bis 40 °C vorgewärmt worden sind, aufgesprüht. Der erhaltene Köder enthielt 2 Gew.-% der Wirkstoffkombination.

Beispiel 2

Auf den nach dem Beispiel 1 hergestellten körnigen Köder wurden 50 g geschmolzenes Adeps solidus von Arzneibuchqualität im geschmolzenen Zustand aufgeblasen. Nach dem Abkühlen erstarrte die Substanz und bildete auf der Oberfläche der Maiskörner einen witterungsbeständigen Schutzüberzug; ausserdem wirkte es im Pansen der Wiederkäuer als schaumzerstörende Substanz. Der Schutzüberzug schmolz bei 36 bis 38 °C (der Körpertemperatur der Tiere) schon im Maul der Tiere von dem Köder ab, das heisst die Wirkstoffkombination wurde vom Organismus ungehindert absorbiert.

Beispiel 3

Es wurde in der in den Beispielen 1 oder 2 beschriebenen Weise gearbeitet, jedoch eine Wirkstoffkombination der folgenden Zusammensetzung verwendet:

80 g 7-Cchlor-1,3-dihydro-1-methyl-5-phenyl-2H-1,4-benzodiazepin-2-on

20 g 1,3-Dihydro-7-nitro-5-phenyl-2H-1,4-benzo-diazepin-2-on

5 g 10-(2-Dimethylamino-n-propyl)-phenothiazin

15 g Ester von N-(β-Hydroxyäthyl)-N'-[3-(3'-γ-chlorphenothiazinyl)-n-propyl]-piperazindifumarat oder diäthylsulfonat mit Trimethoxybenzoesäure.

Beispiel 4

Es wurde in der in den Beispielen 1 oder 2 beschriebenen Weise gearbeitet, jedoch eine Wirkstoffkombination der folgenden Zusammensetzung verwendet:

120 g 1,3-Dihydro-7-nitro-5-phenyl-2H-1,4-benzodiazepin-2-on

7,5 g 10-(2-Dimethylamino-n-propyl)-phenothiazin

10 g Ester von N-(β-Hydroxyäthyl)-N'-[3-(3'-γ-chlorphenothiazinyl)-n-propyl]-piperazin-difumarat oder -diäthylsulfonat mit Trimethoxybenzoesäure.

Beispiel 5

Es wurde in der in den Beispielen 1 oder 2 beschriebenen Weise gearbeitet, jedoch eine Wirkstoffkombination der folgenden Zusammensetzung verwendet:

50 g 1,3-Dihydro-7-nitro-5-phenyl-2H-1,4-benzo-diazepin-2-on

5 g 5-(1'-Cyclohexenyl)-1,5-dimethyl-barbitursäure

80 g 7-Chlor-2-methylamino-5-phenyl-3H-1,4-benzodiazepin-4-oxyd

7,5 g Ester von N-(β-Hydroxyäthyl)-N'-[3-(3'-γ-chlorphenothiazinyl)-n-propyl]-piperazin-difumarat oder -diäthylsulfonat mit Trimethoxybenzoesäure.

**Beispiel 6**

Es wurde in der in den Beispielen 1 oder 2 beschriebenen Weise gearbeitet, jedoch eine Wirkstoffkombination der folgenden Zusammensetzung verwendet.

8 g 5,5-Diallylbarbitursäure
15 g 2-Chlor-10-(3'-dimethylamino-n-propyl)-
phenothiazin
90 g 1-(3,4-Dimethoxyphenyl)-4-methyl-5-äthyl-
7,8-dimethoxy-5H-2,3-benzodiazepin.

Mit den in den Beispielen beschriebenen Ködern wurden bei ihrer erfindungsgemässen Verwendung in den Jahren 1979/1980 insgesamt 1028 Stück Grosswild (985 Damhirsche, 3 Rehe, 34 Rothirsche und 16 Wildschweine) eingefangen. Von den eingefangenen Tieren gingen nur 2 bis 4% zugrunde.

**Patentansprüche für die Vertragsstaaten: BE, CH, DE, FR, GB, IT, LI, LU, NL, SE**

1. Köder zum Einfangen von wild lebenden Säugetieren, gegebenenfalls mit einem Gehalt an einem schwächer wirkenden Tranquillantium, dadurch gekennzeichnet, dass sie 1 oder mehr als Futter für das Wild geeignete[n] Futterstoff(e) und auf der Oberfläche des Futterstoffes beziehungsweise der Futterstoffe mindestens 0,5 Gew.-% einer Wirkstoffkombination, bestehend aus
   a) 35 bis 95 Gew.-% von 1 oder mehr Schlafmittel(n) unter Ausschluss von Barbituraten und/oder schwächer wirkenden Tranquillantium beziehungsweise Tranquillantia und
   b) 5 bis 35 Gew.-% von 1 oder mehr stark wirkenden Tranquillantium beziehungsweise Tranquillantia und gegebenenfalls
   c) bis zu 20 Gew.-% von 1 oder mehr Substanz(en) mit Antihistaminwirkung und/oder gegebenenfalls
   d) bis zu 10 Gew.-% von 1 oder mehr Antiepilepticum beziehungsweise Antiepileptica,
bezogen auf das Gewicht des Futterstoffes beziehungsweise der Futterstoffe, sowie gegebenenfalls 1 oder mehr an sich bekannte[n] Hilfsstoff(e), zweckmässig Träger, die Haftung der Wirkstoffkombination, den Geschmack und/oder die Witterungsbeständigkeit verbessernde[n] Stoff(e) und/oder Farbstoff(e) enthalten.

2. Köder nach Anspruch 1, insbesondere zum Einfangen von wiederkäuendem Grosswild, dadurch gekennzeichnet, dass sie die Wirkstoffkombination in einer Menge von höchstens 7 Gew.-%, bezogen auf das Gewicht des Futterstoffes beziehungsweise der Futterstoffe, enthalten.

3. Köder nach Anspruch 1, insbesondere zum Einfangen von Wildarten mit ungeteiltem Magen, dadurch gekennzeichnet, dass sie die Wirkstoffkombination in einer Menge von höchstens 10 Gew.-%, bezogen auf das Gewicht des Futterstoffes beziehungsweise der Futterstoffe, enthalten.

4. Köder nach Anspruch 1 bis 3, dadurch gekennzeichnet, dass sie die Wirkstoffkombination in einer Menge von 1 bis 5 Gew.-%, bezogen auf das Gewicht des Futterstoffes beziehungsweise der Futterstoffe, enthalten.

5. Köder nach Anspruch 1 bis 4, dadurch gekennzeichnet, dass sie die Wirkstoffkombination in einer Menge von 2 bis 4 Gew.-%, bezogen auf das Gewicht des Futterstoffes beziehungsweise der Futterstoffe, enthalten.

6. Köder nach Anspruch 1 bis 5, dadurch gekennzeichnet, dass sie als Hilfsstoff Adeps solidus enthalten.

7. Köder nach Anspruch 1 bis 6, dadurch gekennzeichnet, dass sie das Adeps solidus in einer Menge von 0,1 bis 120 Gew.-%, bezogen auf das Gewicht der Wirkstoffkombination, enthalten.

8. Köder nach Anspruch 1 bis 7, dadurch gekennzeichnet, dass sie als Futterstoff(e) körniges Futter, insbesondere Getreidekörner und/oder granuliertes Futter, enthalten.

9. Verfahren zur Herstellung der Köder nach Anspruch 1 bis 8, dadurch gekennzeichnet, dass man in an sich bekannter Weise in 1 oder mehr Schritten die Wirkstoffkombination und gegebenenfalls den beziehungsweise die Hilfsstoff(e) auf die Oberfläche des Futterstoffes beziehungsweise der Futterstoffe aufbringt.

10. Verfahren nach Anspruch 9, dadurch gekennzeichnet, dass man im Falle der Verwendung von Adeps solidus als Träger und/oder die Witterungsbeständigkeit verbesserndem Hilfsstoff dieses in einem gesonderten Schritt auf die Oberfläche des beziehungsweise der die Wirkstoffkombination enthaltenden Futterstoffe[s] aufbringt.

11. Verwendung der Köder nach Anspruch 1 bis 8 zum Einfangen von wild lebenden Säugetieren durch ihr Auslegen an Stellen, an welchen das Wild Nahrung zu sich nimmt, und sonstigen von ihm öfter aufgesuchten Stellen mit darauffolgendem Einsammeln des schlafenden oder betäubten Wildes an der Stelle des ausgelegten Köders oder in deren Umgebung.

12. Verwendung nach Anspruch 11, dadurch gekennzeichnet, dass man das Auslegen der Köder in der Wildart entsprechenden Portionen, beim Fangen von Grosswild zweckmässig in Portionen von 50 bis 300 g, vornimmt.

**Patentansprüche für den Vertragsstaat: AT**

1. Verfahren zur Herstellung von Ködern zum Einfangen von wild lebenden Säugetieren, gegebenenfalls mit einem Gehalt an einem schwächer wirkenden Tranquillantium, dadurch gekennzeichnet, dass man in an sich bekannter Weise in 1 oder mehr Schritten auf die Oberfläche von 1 oder mehr als Futter für das Wild geeignete[n] Futterstoff(en) mindestens 0,5 Gew.-% einer Wirkstoffkombination, bestehend aus
   a) 35 bis 95 Gew.-% von 1 oder mehr Schlafmittel(n) unter Ausschluss von Barbituraten und/oder schwächer wirkenden Tranquillantium beziehungsweise Tranquillantia und
   b) 5 bis 35 Gew.-% von 1 oder mehr stark wirkenden Tranquillantium beziehungsweise Tranquillantia und gegebenenfalls
   c) bis zu 20 Gew.-% von 1 oder mehr Substanz(en) mit Antihistaminwirkung und/oder gegebenenfalls

d) bis zu 10 Gew.-% von 1 oder mehr Antiepilepticum beziehungsweise Antiepileptica,
bezogen auf das Gewicht des Futterstoffes beziehungsweise der Futterstoffe, sowie gegebenenfalls 1 oder mehr an sich bekannte[n] Hilfsstoff(e), zweckmässig Träger, die Haftung der Wirkstoffkombination, den Geschmack und/oder die Witterungsbeständigkeit verbessernde[n] Stoff(e) und/oder Farbstoff(e) aufbringt.

2. Verfahren nach Anspruch 1, insbesondere zur Herstellung von Ködern zum Einfangen von wiederkäuendem Grosswild, dadurch gekennzeichnet, dass man die Wirkstoffkombination in einer Menge von höchstens 7 Gew.-%, bezogen auf das Gewicht des Futterstoffes beziehungsweise der Futterstoffe, verwendet.

3. Verfahren nach Anspruch 1, insbesondere zur Herstellung von Ködern zum Einfangen von Wildarten mit umgeteiltem Magen, dadurch gekennzeichnet, dass man die Wirkstoffkombination in einer Menge von höchstens 10 Gew.-%, bezogen auf das Gewicht des Futterstoffes beziehungsweise der Futterstoffe, verwendet.

4. Verfahren nach Anspruch 1 bis 3, dadurch gekennzeichnet, dass man die Wirkstoffkombination in einer Menge von 1 bis 5 Gew.-%, bezogen auf das Gewicht des Futterstoffes beziehungsweise der Futterstoffe, verwendet.

5. Verfahren nach Anspruch 1 bis 4, dadurch gekennzeichnet, dass man die Wirkstoffkombination in einer Menge von 2 bis 4 Gew.-%, bezogen auf das Gewicht des Futterstoffes beziehungsweise der Futterstoffe, verwendet.

6. Verfahren nach Anspruch 1 bis 5, dadurch gekennzeichnet, dass man als Hilfsstoff Adeps solidus verwendet.

7. Verfahren nach Anspruch 1 bis 6, dadurch gekennzeichnet, dass man das Adeps solidus in einer Menge von 0,1 bis 120 Gew.-%, bezogen auf das Gewicht der Wirkstoffkombination, verwendet.

8. Verfahren nach Anspruch 1 bis 7, dadurch gekennzeichnet, dass man als Futterstoff(e) körniges Futter, insbesondere Getreidekörner und/oder granuliertes Futter, verwendet.

9. Verfahren nach Anspruch 1 bis 8, dadurch gekennzeichnet, dass man im Falle der Verwendung von Adeps solidus als Träger und/oder die Witterungsbeständigkeit verbesserndem Hilfsstoff dieses in einem gesonderten Schritt auf die Oberfläche des beziehungsweise der die Wirkstoffkombination enthaltenden Futterstoffe[s] aufbringt.

**Claims for the Contracting States: BE, CH, DE, FR, GB, IT, LI, LU, NL, SE**

1. Baits for catching mammals living in the wild state, optionally having a content of a tranquilizer having a weaker activity, characterized in that they contain 1 or more food material(s) suitable as [a] food for the game and on the surface of the food material or food materials, respectively, at least 0.5% by weight of a combination of active principles, consisting of
a) from 35 to 95% by weight of 1 or more soporific(s) with the exception of barbiturates and/or tranquilizer or tranquilizers, respectively, having a weaker activity and
b) from 5 to 35% by weight of 1 or more tranquilizer or tranquilizers, respectively, having a strong activity and optionally
c) up to 20% by weight of 1 or more substance(s) having an antihistaminic activity and/or optionally
d) up to 10% by weight of 1 or more antiepileptic or antiepileptics, respectively,
referred to the weight of the food material or food materials, respectively, as well as optionally 1 or more auxiliary material(s) known as such, usefully carrier(s), material(s) improving the adhesion of the combination of active principles, the flavour and/or the resistance to the weather and/or dye stuff(s).

2. Baits according to claim 1, particularly for catching ruminant big game, characterized in that they contain the combination of active principles in an amount of at most 7% by weight, referred to the weight of the food material or food materials, respectively.

3. Baits according to claim 1, particularly for catching game species having an undivided stomach, characterized in that they contain the combination of active principles in an amount of at most 10% by weight, referred to the weight of the food material or food materials, respectively.

4. Baits according to claims 1 to 3, characterized in that they contain the combination of active principles in an amount of from 1 to 5% by weight, referred to the weight of the food material or food materials, respectively.

5. Baits according to claims 1 to 4, characterized in that, that they contain the combination of active principles in an amount of from 2 to 4% by weight, referred to the weight of the food material or food materials, respectively.

6. Baits according to claims 1 to 5, characterized in that they contain as an auxiliary material adeps solidus.

7. Baits according to claims 1 to 6, characterized in that they contain the adeps solidus in an amount of from 0.1 to 120% by weight, referred to the weight of the combination of active principles.

8. Baits according to claims 1 to 7, characterized in that they contain as food material(s) granular food, particularly cereal grains and/or granulated food.

9. A process for preparing the baits according to claims 1 to 8. characterized in that in a manner known per se one applies in 1 or more steps the combination of active principles and optionally the auxiliary material(s) to the surface of the food material or food materials, respectively.

10. A process according to claim 9, characterized in that in case of using adeps solidus as a carrier and/or an auxiliary material improving the resistance to weather one applies this to the surface of the food material(s) containing the combination of active principles in a separate step.

11. The use of the baits according to claims 1 to 8 for catching mammals living in the wild state by laying it out at places at which the game takes food and other places looked up by it frequently and subsequently collecting the sleeping or anaesthetized game at the place of the bait layed out or in its environment.

12. The use according to claim 11, characterized in that one carries out the laying out of the bait in portions corresponding to the game species, in case of catching big game suitably in portions of 50 to 300 g.


**Claims for the Contracting State: AT**

1. A process for the preparation of baits for catching mammals living in the wild state, optionally having a content of a tranquilizer having a weaker activity, characterized in that in a manner known per se one applies in 1 or more steps to the surface of 1 or more food material(s) suitable as [a] food for the game at least 0.5% by weight of a combination of active principles, consisting of

a) from 35 to 95% by weight of 1 or more soporific(s) with the exception of barbiturates and/or tranquilizer or tranquilizers, respectively, having a weaker activity and

b) from 5 to 35% by weight of 1 or more tranquilizer or tranquilizers, respectively, having a strong activity and optionally

c) up to 20% by weight of 1 or more substance(s) having an antihistaminic activity and/or optionally

d) up to 10% by weight of 1 or more antiepileptic or antiepileptics, respectively,
referred to the weight of the food material or food materials, respectively, as well as optionally 1 or more auxiliary material(s) known as such, usefully carrier(s), material(s) improving the adhesion of the combination of active principles, the flavour and/or the resistance to the weather and/or dye stuff(s).

2. A process according to claim 1, particularly for the preparation of baits for catching ruminant big game, characterized in that one uses the combination of active principles in an amount of at most 7% by weight, referred to the weight of the food material or food materials respectively.

3. A process according to claim 1, particularly for the preparation of baits for catching game species having an undivided stomach, characterized in that one uses the combination of active principles in an amount of at most 10% by weight, referred to the weight of the food material or food materials, respectively.

4. A process according to claims 1 to 3, characterized in that one uses the combination of active principles in an amount of from 1 to 5% by weight, referred to the weight of the food material or food materials, respectively.

5. A process according to claims 1 to 4, characterized in that one uses the combination of active principles in an amount of from 2 to 4% by weight, referred to the weight of the food material or food materials, respectively.

6. A process according to claims 1 to 5, characterized in that one uses as an auxiliary material adeps solidus.

7. A process according to claims 1 to 6, characterized in that one uses the adeps solidus in an amount of from 0.1 to 120% by weight, referred to the weight of the combination of active principles.

8. A process according to claims 1 to 7, characterized in that one uses as food material(s) granular food, particularly cereal grains and/or granulated food.

9. A process according to claims 1 to 8, characterized in that in case of using adeps solidus as a carrier and/or an auxiliary material improving the resistance to weather one applies this to the surface of the food material(s) containing the combination of active principles in a separate step.

**Revendications pour les Etats Contractants: BE, CH, DE, FR, GB, IT, LI, LU, NL, SE**

1. Appâts pour prendre des mammifères sauvages vivants, contenant éventuellement un tranquillisant à action plus faible, caractérisés en ce qu'ils contiennent un ou plusieurs produits alimentaires appropriés comme aliments pour les animaux sauvages et à la surface du ou des produit(s) alimentaire(s) au moins 0,5% en poids d'une combinaison de substances actives, constituée de

a) 35 à 95% en poids d'un ou plusieurs somnifères, à l'exclusion des barbituriques, et/ou tranquillisant(s) à action plus faible et

b) 5 à 35% en poids d'un ou plusieurs tranquillisant(s) à action forte et éventuellement

c) jusqu'à 20% en poids d'une ou plusieurs substance(s) à action antihistaminique et/ou éventuellement

d) jusqu'à 10% en poids d'un ou plusieurs antiépileptique(s)y par rapport au poids du ou des produit(s) alimentaire(s), ainsi qu'éventuellement un ou plusieurs additif(s) connu(s) en pratique des supports qui contiennent des corps qui améliorent la fixation de la combinaison de substances actives, le goût et/ou la stabilité aux intempéries et/ou un ou plusieurs colorant(s).

2. Appâts selon la revendication 1, en particulier pour prendre du gros gibier ruminant, caractérisés en ce qu'ils contiennent la combinaison de substances actives en une quantité d'au plus 7% en poids par rapport au poids du ou des produit(s) alimentaire(s).

3. Appâts selon la revendication 1, en particulier pour prendre des espèces de gibier ayant un estomac non divisé, caractérisés en ce qu'ils contiennent la combinaison de substances actives en une quantité d'au plus 10% en poids par rapport au poids du ou des produit(s) alimentaire(s).

4. Appâts selon les revendications 1 à 3, caractérisés en ce qu'ils contiennent la combinaison de substances actives en une quantité de 1 à 5% en poids par rapport au poids du ou des produit(s) alimentaire(s).

5. Appâts selon les revendications 1 à 4, caractérisés en ce qu'ils contiennent la combinaison de substances actives en une quantité de 2 à 4% en poids par rapport au poids du ou des produit(s) alimentaire(s).

6. Appâts selon les revendications 1 à 5, caractérisés en ce qu'ils contiennent comme additif de la graisse solide (Adeps solidus).

7. Appâts selon les revendications 1 à 6, caractérisés en ce qu'ils contiennent de la graisse solide (Adeps solidus) en une quantité de 0,1 à 120% en poids par rapport au poids de la combinaison de substances actives.

8. Appâts selon les revendications 1 à 7, caractérisés en ce qu'ils contiennent comme produit(s) alimentaire(s) un aliment en grains, en particulier des graines de céréales et/ou un aliment granulé.

9. Procédé de préparation des appâts selon les revendications 1 à 8, caractérisé en ce qu'on dépose à la surface du ou des produit(s) alimentaire(s) de façon connue en une ou plusieurs étapes la combinaison de substances actives et éventuellement le ou les additif(s).

10. Procédé selon la revendication 9, caractérisé en ce que, au cas où l'on utilise de la graisse solide (Adeps solidus) comme support et/ou additif améliorant la stabilité aux intempéries on dépose cet additif en une étape particulière à la surface du ou des produit(s) alimentaire(s) contenant la combinaison de substances actives.

11. Application des appâts selon les revendications 1 à 8 pour prendre les mammifères sauvages vivants en les disposant à des endroits où les animaux prennent leur nourriture et à d'autres endroits qu'ils fréquentent très souvent en recueillant ensuite les animaux endormis ou assoupis aux endroits où les appâts ont été disposés ou à proximité.

12. Application selon la revendication 11, caractérisée en ce qu'on procède au dépôt des appâts en portions correspondant aux animaux sauvages, lorsqu'on prend du gros gibier en pratique en portions de 50 à 300 g.

**Revendications pour l'Etat contractant: AT**

1. Procédé de préparation d'appâts pour prendre des mammifères sauvages vivants, contenant éventuellement un tranquillisant à action plus faible caractérises en ce qu'on dépose à la surface du ou des produit(s) alimentaire(s) de façon connue en une ou plusieurs étapes un ou plusieurs produits alimentaires appropriés comme aliments pour les animaux sauvages au moins 0,5% en poids d'une combinaison de substances actives, constituée de

a) 35 à 95% en poids d'un ou plusieurs somnifères, à l'exclusion des barbituriques, et/ou tranquillisant(s) à action plus faible et

b) 5 à 35% en poids d'un ou plusieurs tranquillisant(s) à action forte et éventuellement

c) jusqu'à 20% en poids d'une ou plusieurs substance(s) à action antihistaminique et/ou éventuellement

d) jusqu'à 10% en poids d'un ou plusieurs antiépileptique(s) par rapport au poids du ou des produit(s) alimentaire(s), ainsi qu'eventuellement un ou plusieurs additif(s) connu(s) en pratique des supports qui contiennent des corps qui améliorent la fixation de la combinaison de substances actives, le goût et/ou la stabilité aux intempéries et/ou un ou plusieurs colorant(s).

2. Procédé selon la revendication 1, en particulier pour prendre du gros gibier ruminant, caractérisés en ce qu'on utilise la combinaison de substances actives en une quantité d'au plus 7% en poids par rapport au poids du ou des produit(s) alimentaire(s).

3. Procédé selon la revendication 1, en particulier pour prendre des espèces de gibier ayant un estomac non divisé, caractérisés en ce qu'on utilise la combinaison de substances actives en une quantité d'au plus 10% en poids par rapport au poids du ou des produit(s) alimentaire(s).

4. Procédé selon les revendications 1 à 3, caractérisé en ce qu'on utilise la combinaison de substances actives en une quantité de 1 à 5% en poids par rapport au poids du ou des produit(s) alimentaire(s).

5. Procédé selon les revendications 1 à 4, caractérisé en ce qu'on utilise la combinaison de substances actives en une quantité de 2 à 4% en poids par rapport au poids du ou des produit(s) alimentaire(s).

6. Procédé selon les revendications 1 à 5, caractérisé en ce qu'on utilise comme additif de la graisse solide (Adeps solidus).

7. Procédé selon les revendications 1 à 6, caractérisé en ce qu'on utilise la graisse solide (Adeps solidus) en une quantité de 0,1 à 120% en poids par rapport au poids de la combinaison de substances actives.

8. Procédé selon les revendications 1 à 7, caractérisé en ce qu'on utilise comme produit(s) alimentaire(s) un aliment en grains, en particulier des graines de céréales et/ou un aliment granule.

9. Procédé selon les revendications 1 à 8, caractérisé en ce que, au cas où l'on utilise de la graisse solide (Adeps solidus) comme support et/ou additif améliorant la stabilité aux intempéries on depose cet additif en une étape particulière à la surface du ou des produit(s) alimentaire(s) contenant la combinaison de substances actives.